# EUROPEAN PATENT APPLICATION

(11) **EP 1 234 508 A1**
(43) Date of publication of application: **28.08.2002**
(21) Application number: 01200614.4
(22) Date of filing: 21.02.2001
(51) Int. Cl.: A23K 1/165, A61K 31/195, A23K 1/16, A61K 31/19, A23K 1/18

(54) **Antiprotozoal methods, compositions and feedstuffs**

(71) Applicant: UNIVERSITEIT GENT, 9000 Gent (BE)
(72) Inventor: Remon, Jean Paul, 9090 Melle (BE); Vermeulen, Brenda, 9140 Temse (BE)
(74) Representative: Bird, William Edward

(57) **Abstract**

Methods and compositions for the prophylactic treatment of coccidiosis and other protozoal infections are described. Preferably, these are administered in the drinking water of the animals, e.g. in the drinking water of poultry to provide a mass prophylactic treatment.

These formulations include L-arginine and may also include a modulator of the NO biosynthesis pathway, e.g. and NSAID such as Ibuprofen.

These formulations have also been found to improve egg production in hens.

## Description

The present invention relates to an antiprotozoal method, particularly a method for prohpylactic control of a parasitic infection of a vertebrate with a protozoan protist (especially those infections caused by microsporidia), and more particularly a method for the prophylactic treatment of coccidiosis in poultry. The invention also relates to feed additives, drinking water additives, husbandry, veterinary or pharmaceutical compositions comprising a therapeutic or effective amount of a basic amino-acid and optionally a non-steroidal anti-inflammatory drug. The compositions may be used, for instance, for the prohpylactic control of a protozoal infection of poultry or may be used to improve egg laying in hens.

### BACKGROUND OF THE INVENTION

Infestation of various vertebrates, including man, with various intracellular parasites belonging to the kingdom protists are well known. Examples of such intracellular parasites include *Trypanosoma gambiense,* an agent of African sleeping sickness and *Entamoeba histolytica*, an agent of amebic dysentery, both belonging to Phylum Mastigophora of protozoan protists; *Plasmodium vivax,* an agent of human malaria belonging to Phylum Apicomplexa of protozoan protists; and *Eimeria,* an agent of coccidiosis namely in poultry such as chickens, or in rabbit, belonging to Phylum Sporozoa of protozoan protists.

Coccidiosis is a devastating protozoan disease of both vertebrates and invertebrates, including man, caused by intra-cellular parasitic protozoa which generally invade the epithelial cells lining the alimentary tract and the cells of associated glands. Because of the crowded conditions under which many domestic animals are raised, it is one of the main concerns of poultry producers and bird keepers in general and is reported both in the developing and the developed world. Its incidence appears to be of greater importance throughout the world than the best known bacterial infections or viral diseases, and it is therefore the cause of significant economic loss throughout the world.

The parasites which infect birds with coccidiosis are from the genus *Eimeria* of the class protozoa and the most economically important species. *Eimeria* species are parasites which are passed from host to host via faeces, containing oocysts. These oocysts live and multiply in the cells of the host, mostly in the intestinal epithelium. Various species of *Eimeria* infect a wide range of hosts, including mammals, but nine species have been recognized as being pathogenic to varying degrees in chickens: *Eimeria tenella, E. acervulina, E. mivati, E. mitis, E. praecox, E. hagani, E. necatrix, E. brunetti* and *E. maxima.*

Although the *Eimeria* are very host-specific, their life cycles are similar. The developmental stages of the avian coccidia can be illustrated by the species *Eimeria tenella* which proliferates in the cecum of the chicken. The life cycle of the *Eimeria* species begins when the host ingests previously sporulated oocysts during ground feeding or by inhalation of dust. Mechanical and chemical action in the intestinal tract ruptures the sporulated oocyst, liberating sporozoites which are carried in the digestive contents and infect various parts of the intestinal tract by penetrating epithelial cells. Subsequent stages involve asexual multiple fission, development of gametes and fertilization to produce a zygote becoming an oocyst which is passed out of the host with the droppings. The oocyst undergoes nuclear and cellular division resulting in the formation of sporozoites which are then sporulated and ingested by a new host. *Eimeria tenella* has received the most attention because it is an extremely pathogenic species, with death occurring on about the fifth day of infection.

Ideally, the best method for combating coccidia is preventive medication. Nowadays, prevention with anticoccidial drugs has significantly changed the evolution of coccidiosis. Such drugs are well known and typically include polyethers (such as monensin, narasin, nicarbazin and mixtures thereof), benzimidazoles, phenylenediamines, quinoxalinediones and triazinetriones. However, some chemotherapeutic drugs cause undesirable side effects. Others lead to the development of reduced sensitivity or resistance to a whole class of chemotherapeutics. Other problems with anticoccidial drugs include excessive drug amounts in the feed causing toxicity in the birds. Further, these chemotherapeutic drugs are mostly expensive compounds which, for this reason, may not be regularly used under all circumstances. Therefore losses due to morbidity from coccidiosis, including reduced weight gains and egg production, and decreased feed conversion, persist.

Immunity from the host against *Eimeria* species is specific to the genus which infected the host. Cross-protection does not occur between different *Eimeria* species. Immunity varies in intensity and duration according to the *Eimeria* species and does not last long if re-infection with the same species does not occur. During infection, different compounds are involved in the development of immunity. As in immune responses to many other parasitic infections of the gastro-intestinal tract, the role of antibodies is at best minor, whereas T-lymphocytes are crucial. Vaccines against avian coccidiosis have been proposed by U.S.Patent No. 5,656,485.

U.S.Patent No. 5,217,997 discloses administering to a mammal about 1 mg to about 1500 mg per day of L-arginine or a pharmaceutically acceptable salt thereof in the treatment of hypertension, bronchial asthma and high vascular disorders such as angina pectoris, cerebral ischemia and preeclampsia of pregnancy.

U.S.Patent No. 5,895,788 discloses treating and/or preventing pulmonary hypertension syndrome (ascites fluid accumulation in the abdominal cavity) in avians by means of drinking water containing a sufficient amount, i.e. from 0.01 weight% up to the water solubility level (about 18 weight% at ambient temperature) of an L-arginine compound (free base or salts). According to this document, broilers with a body weight of about 500 g usually drink about 0.7 liter during their third week and are given drinking water supplemented with 3.0 to 18.4 g/l arginine free base or hydrochloride salt, i.e. a treating amount of at least 600 mg arginine per kg bodyweight per day.

U.S.Patents No. 5,985,336 and 5,976,580 disclose enhancing livability, cumulative weight gain or feed conversion efficiency of poultry by making available to poultry hatchlings for consumption *ad libitum,* before they are offered dry feed *ad libitum,* a high moisture material which does not release free water in an amount sufficient to dampen said hatchlings and which contains, on a percent weight basis of the total composition, (i) at least 40% water, (ii) at least 8% carbohydrates, (iii) at least 7% of an amino acid source such as arginine, and (iv) up to 5% fat. The amount of such a high moisture material fed to chicks should be at least 5 grams until 2 days, 10 grams from 2 to 3 days, and 20 grams from 4 to 7 days.

Several prior art documents teach combinations of ibuprofen (2-(4-isobutylphenyl)propionic acid) and L-arginine. In particular, GB-A-2,193,093 discloses compositions containing 33-46 weight % free ibuprofen, 34-51 weight % L-arginine and 9-29 weight % sodium bicarbonate, the molar ratio of L-arginine to ibuprofen being in the range of 1.1:1 to 1.5:1.

U.S.Patent No. 5,262,179 discloses a water-soluble ibuprofen arginine or lysine salt for use in non-effervescent water-soluble compositions, apparently intended for humans, for the treatment of pain especially when associated with inflammation such as in rheumatic disease.

U.S.Patent No. 5,939,455 discloses that butyric acid salts, among which arginine and lysine butyrates, are used for treating, preventing or ameliorating protozoan infections and further discloses a pharmaceutical composition for potentiating the therapeutic activity of a butyric acid salt, the said composition comprising an effective amount of an inhibitor of β-oxidation of fatty acids such as for instance ibuprofen.

According to U.S.Patent No. 6,051,557, patients receiving or about to receive one or more non-steroidal anti-inflammatory drugs (NSAID) are at risk of developing a malfunctioning of the upper gastrointestinal tract. This irritability of the mucosa of the mouth and of gastric mucosa as a result of oral administration of ibuprofen, a drug with analgesic and anti-inflammatory activity, is further confirmed by U.S.Patent No. 4,689,218. Therefore, use of a NSAID such as ibuprofen should not be expected in the therapy of coccidiosis if such NSAID would possibly induce intestinal mucosal damage which might make coccidiosis much worse.

As a summary, there is a need in the art for an effective method for controlling a parasitic infection of a vertebrate with a protozoan protist, and more particularly a method for the prophylactic treatment of coccidiosis in poultry, which avoids the use of high amounts of possibly toxic chemotherapeutic drugs. Ideally, egg laying should be as normal or even enhanced.

### SUMMARY OF THE INVENTION

As a response to the above-mentioned technical problem, the present invention first provides the use of a basic amino-acid or amino-acid derivative in mass medication of vertebrates (e.g. in feed or drinking water) for the prophylactic treatment of a parasitic infection of a vertebrate with a protozoan protist. A second object of the present invention is the use of a basic amino-acid or amino-acid derivative for the manufacture of a medicine (both for veterinary and pharmaceutical purpose) for the prophylactic treatment of a parasitic infection with a protozoan protist in a mammal, including non-human animals. Another object of the present invention is a feed composition for vertebrates comprising an antiprotozoal prophylactic amount of a basic amino-acid or amino-acid derivative and optionally an alimentary acceptable carrier. A further object of the present invention is a veterinary composition for vertebrates comprising a antiprotozoal prophylactic amount of a basic amino-acid or amino-acid derivative and optionally a veterinary acceptable carrier. Yet another object of the present invention is a pharmaceutical composition comprising an antiprotozoal prophylactic amount of a basic amino-acid or amino-acid derivative and optionally a pharmaceutically acceptable carrier.

In the above-referred uses and compositions:
- a combination of an effective amount of a non-steroidal anti-inflammatory drug and of a basic amino-acid or amino-acid derivative may be used instead of the said basic amino-acid or amino-acid derivative alone.
- the basic amino-acid preferably is a naturally-occurring basic amino-acid such as histidine, lysine or, more preferably, arginine.
- when a non-steroidal anti-inflammatory drug is used in combination with the basic amino-acid or amino-acid derivative, the said non-steroidal anti-inflammatory drug can be an acid such as ibuprofen (2-(4-isobutylphenyl)propionic acid), and the combination can be a salt of the acid non-steroidal anti-inflammatory drug and of the basic amino-acid such as for instance the arginine or lysine salt of ibuprofen. Under the same conditions, the veterinary and pharmaceutical compositions preferably comprise a veterinary or pharmaceutically acceptable carrier for the said non-steroidal anti-inflammatory drug.
- the parasitic infection to be prophylactically treated is preferably coccidiosis.
- the composition is preferably in the form of a drinkable formulation.
- the antiprotozoal amount preferably is an oocyst production limiting amount, or an amount which stimulates the production of reactive oxygen intermediates and/or reactive nitrogen intermediates in the body of the vertebrate to which the composition is administered, or a nitrogen oxide synthetase inducing amount.

The present invention also includes the use of a basic amino acid or a derivative thereof in a drinking water formulation for the enhancement of egg production in poultry. The present invention also includes a drinking water composition comprising a basic amino acid or a derivative thereof for the enhancement of egg production in poultry. Eggs are laid earlier by the hens and the egg weights are increased.

### BRIEF DESCRIPTION OF THE DRAWINGS

Figure 1 shows the influence of the combination ibuprofen - arginine and of L-arginine compounds as a prevention means on lesion scores after an experimentally induced coccidiosis infection.
Figure 2 shows the influence of the combination ibuprofen - arginine and of L-arginine compounds as a prevention means on the number of oocysts in faecal samples after an experimentally induced coccidiosis infection.
Figure 3 shows the influence of the combination ibuprofen - arginine and of L-arginine hydrochloride on lesion scores after an experimentally induced coccidiosis infection with *E. acervulina* oocysts following a primary infection.
Figure 4 shows the production of a reactive intermediaries after induction of nitrogen oxide synthase after the release of IFN-γ and TNF-α stimulated by the incidence of coccidiosis.
Figure 5 is a graph showing the number of eggs laid by a control group (n =10) (▲) and laid by a group of laying hens (n=10) treated with a drinking water formulation containing L-arginine (15 mg/kg) (■).
Figure 6 is a graph of the mean weight (g) of at least 3 eggs laid by a control group (n =10) (▲) and laid by a group of laying hens (n=10) treated with a drinking water formulation containing L-arginine (15 mg/kg) ( ■ ).

### DETAILED DESCRIPTION OF THE INVENTION

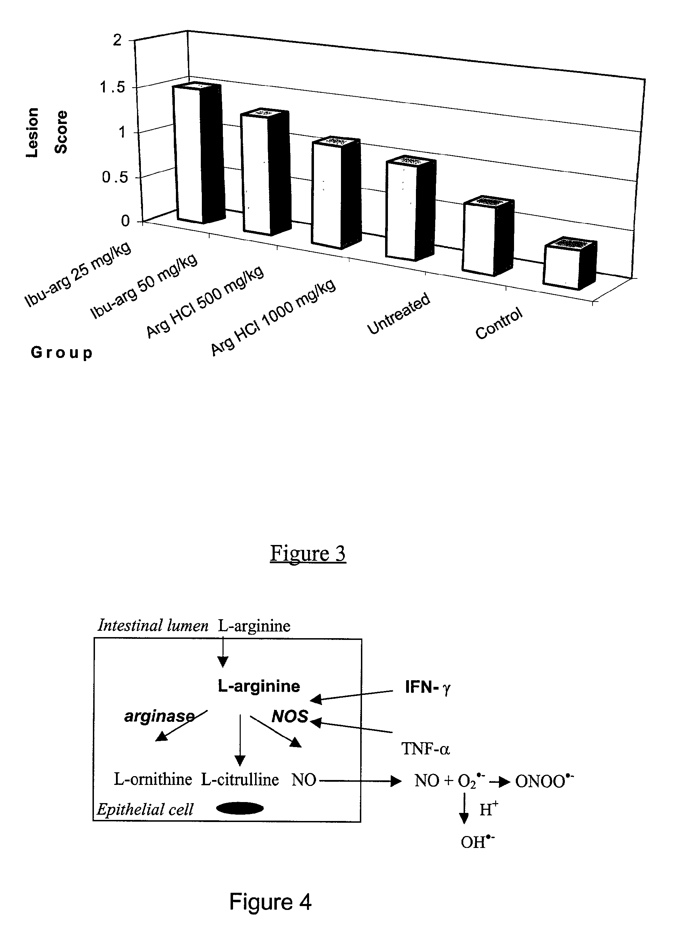

Without wishing to be bound by any theory, it is presently believed that the invention may be explained as follows. Cytokines such as interferon-γ (IFN-γ) and tumor necrosis factor-α (TNF-α) are produced in case of inflammations and infections and are involved in the regulation of mucosal immunity and functions of epithelial cells, thus being effective against intracellular parasites. IFN-γ inhibits growth and development of parasites by inducing many different cellular changes having deleterious actions on the parasite. TNF-α mediates immune responses, which limit oocyst production. IFN-γ and TNF-α act synergistically in many functions, including the stimulation of reactive oxygen intermediates (ROIs) production. ROIs are destructive compounds produced after the stimulation of leukocytes by parasite antigens. When leukocytes are stimulated by parasite antigens, oxygen-uptake increases and molecular oxygen is reduced to the superoxide anion (•O₂⁻), which can go on to form a hydroxyl radical (•OH⁻) and hydrogen peroxide (H₂O₂) in the presence of protons. Since *Eimeria* species are sensitive to ROIs, the latter are able to kill intra- and extracellular stages of *Eimeria* species. Furthermore IFN-γ and TNF-α are involved in the production of reactive nitrogen intermediates (RNIs) by inducing/stimulating the activity of nitrogen oxide synthetase (NOS). NOS activity is usually so low that arginase converts most of the cellular L-arginine, taken up from the intestinal lumen, to L-ornithine. IFN-γ and TNF-α induce a form of NOS (iNOS), which results in a large increase in the amount of L-arginine that is converted to L-citrulline with an associated nitric oxide (NO) production. NO can also interact with superoxide anion (•O₂⁻) to form the potent oxidant peroxynitrite (ONOO⁻) or the highly reactive hydroxyl radical (•OH⁻) in the presence of protons, as represented on the scheme of Fig. 4.

*Eimeria* species are sensitive to these reactive nitrogen intermediates and therefore IFN-γ and TNF-α inhibit the development of *Eimeria* species by the induction of NOS. Production of NO as a result of infection can be considered as a part of the inflammatory response because it is most often triggered by inflammatory cytokines such as IFN-γ and TNF-α. Because inflammatory immune responses divert energy from growth, they may adversely affect weight gain, the major contributor to the production losses that accompany coccidiosis infections. In a similar way, NO is thought to be an important defense mechanism against other intracellular parasites such as *Trypanosoma* and *Plasmodia* species cited hereinabove.

NO is normally produced in small amounts by constitutive NOS (cNOS) and is involved in a lot of physiological processes in the cardiovascular and respiratory system. During an infection or inflammation, large amounts of NO are produced by an induced NOS (iNOS). Nitrate (NO₃⁻) and nitrite (NO₂⁻) ions are stable metabolites arising from NO during its reaction with other compounds such as the superoxide anion (•O₂⁻). These metabolites can be used as indicia for an *in vivo* response to infections. Literature describes significantly higher plasma concentrations of nitrate and nitrite ions after primary infections of chickens with *Eimeria* species. Besides, a maximal concentration of both ions in plasma is reported when mucosal lesions and excretion of oocysts are maximal.

Because of the absence of the ornithine-cycle in chickens, the latter cannot synthesize L-arginine and are dependent upon exogenous sources such as diet for the metabolic utilization of L-arginine. L-arginine is an essential amino-acid for chickens as well as a substrate for the biosynthesis of nitric oxide (NO). Biosynthesis of NO by means of iNOS can be stimulated during immune responses to infections. Therefore, production of NO as a response to an infection such as coccidiosis and its subsequent effects on the host depends on levels of L-arginine.

When a basic amino-acid or amino-acid derivative such as arginine is administered after a coccidiosis infection, the induced NOS enzyme (iNOS) - activated during infections and inflammations - can already be saturated and the supplementary administration of such amino-acid has no effect on the production of NO.

Administration of the antiprotozoal active compounds of the invention in the form of drinkable formulations, particularly drinking water, is economically favorable to treat a large group of animals. Advantages of drinking water medication are the easiness of administration, the reproducible and controllable dosage of the formulation and the low cost price of the alimentary acceptable carrier. Consequently, the preferred antiprotozoal active compounds and optionally non-steroidal anti-inflammatory drugs used in this invention are water-soluble to some extent or can be made water-dispersible by well known techniques. However, the term "feed" as used herein is not limited to drinking formulations but also includes solid feed medications as are already known in the art.

The term "amino-acid derivative" as used herein includes any salt that may be formed between the basic amino-acid and an acidic moiety. Preferably the said salt is not a butyric acid salt. Suitable anions for such salts include halides (such as fluoride, chloride, bromide and iodide), borate, hypobromite, hypochlorite, nitrite, nitrate, hyponitrite, sulfate, disulfate, sulfite, sulfonate, phosphate, diphosphate, phosphite, phosphonate, diphosphonate, perchlorate, perchlorite, carbonate, bicarbonate, oxalate, malonate, succinate, lactate, acetate, benzoate, citrate, tosylate, permanganate, manganate, chromate, dichromate, orthosilicate, metasilicate, cyanate, isocyanate, thiocyanate and the like. Suitable cations for such salts include hydrogen and alcaline metals such as potassium and sodium.

Ibuprofen, 2-(-4-isobutylphenyl)-propionic acid, is widely used in veterinary medicine. Ibuprofen is completely insoluble in water and therefore a water-dispersible or water-soluble form thereof such as a salt thereof with L-arginine-an antiprotozoal compound of the invention - is preferably produced for the administration of a drinking water formulation containing ibuprofen. Doses ranging from about 0.1 to about 50 mg ibuprofen per kg bodyweight per day can safely be administered for the prophylactic treatment of broiler chickens.

Other non-steroidal anti-inflammatory drugs may be used, alternatively to ibuprofen, in combination with a basic amino-acid or amino-acid derivative for the performance of the present invention. Suitable non-steroidal anti-inflammatory drugs for this purpose include acetyl salicylic acid; derivatives of aryl acetic acid such as aceclofenac, bufexamac, diclofenac, sulindac, sodium diclofenac, tolmetine; aryl proprionic acid derivatives such as ibuprofen, naproxen, sodium naproxen, flurbiprofen, fenoprofen, pirprofen, ketoprofen, tiaprofenic acid carprofen, vedaprofen; indole derivatives such as indomethacine, proglumetacine; phenamates including niflumic acid; oxicams such as piroxicam, meloxicam, tenoxicam; nimesulide; flunixine.

Arginine, i.e. 2-amino-5-guanidinovaleric acid, is a basic amino acid with a positively charged guanidine group and is the preferred basic amino-acid for performing the present invention. L-arginine free base or a salt thereof can be safely administered for the prophylactic treatment of broiler chickens in a feed medication, especially a drinking water medication. The preferred amount of the L-arginine derivative used in the present invention will depend upon the specific form of the said derivative. Generally, L-arginine free base or a L-arginine salt such as hydrochloride may be given daily in a dose of about 5 - 200 mg up to 5 to about 1000 mg per kg bodyweight per day of the vertebrate to be treated. Importantly, it will be noted that such amounts are far below the amounts needed for treating and/or preventing pulmonary hypertension syndrome in avians such as disclosed by U.S.Patent No. 5,895,788. The ratio of ibuprofen to arginine can be varied between 1:1 and 1:1000.

In drinking water medication, it is preferred to adjust the pH within a range of about 5.2 to 7 for instance by the addition of an acid. Suitable acids for this purpose include phosphoric acid and low molecular weight carboxylic acids having up to 10 carbon atoms, more preferably from 2 to 5 carbon atoms, such as citric acid, propionic acid and fumaric acid. The amount of such pH adjusting acids usually range from about 0.5% to about 5% by weight.

Vertebrates which can be submitted to the prophylactic treatment in accordance with the present invention include in particular mammals and avians but also fishes and reptiles. Among avians, chickens and turkeys are the specied most commonly treated for coccidiosis, but the present invention can also be used with other poultry species such as ducks, geese, quail, pheasants and ostriches. Among mammals, rabbits and man are most commonly treated for coccidiosis, but the present invention can also be used with other stock farming animals such as sheep, cattle, pigs etc.

Without being limited by theory it is believed that the use of an NSAID has an effect as a modulator of the NO synthesis pathway. The enzyme cyclooxygenase (COX) is believed to be in competitive interaction with inducible nitrogen oxide synthetase (iNOS). COX catalytically active in the conversion of arachidonic acid to prostaglandines. Its structure is similar to that of iNOS and it is believed to compete with iNOS for available L-arginine thus removing or limiting this for conversion into NO. NSAID's are able to inhibit COX thus they modulate improvement of the availablity of L-arginine. The inhibition of the COX can be achieved by increasing the bioavailability of arginine - this can be achieved by using arginine in drinking water and/or by the use of an NSAID as inhibitor of COX. Other inhibitors of COX may be used in place of an NSAID.

The veterinary and pharmaceutical compositions of the present invention may comprise a veterinary or pharmaceutically acceptable carrier. Suitable carriers for this purpose are well known to those skilled in the art and there is no particular restriction to their selection within the present invention. Such carriers typically include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems such as liposomes, microspheres, microemulsions, nanoparticles, nanocapsules and so on. Depending on the route of administration, the pharmaceutical or veterinary composition comprising the active ingredient may additionally require protective coatings well known in the art. When the protozoan protist against which the prophylactic treatment is directed infects the intestinal tract, the pharmaceutical or veterinary composition of the invention should preferably be administered in a way to directly reach the intestinal tract, i.e. orally.

During coccidiosis infections, the body weight gain of poultry is decreased because of a damage of the epithelial cells of the intestines. Lesions of the intestines caused by the different *Eimeria* species are specific and may be scored by the method developed by Johnson and Reid as disclosed for instance in *Experimental parasitology* (1970) 28:30-36. Furthermore, microscopic sections of the intestines of chickens were performed in order to identify the *Eimeria* species and to confirm results of the scoring method. Finally, the oocysts of the *Eimeria* species were localized in the faeces where they were determined quantitatively. When ibuprofen had an anticoccidial effect or when NO - synthesized after administration of L-arginine - was toxic for the *Eimeria* species, then a decrease in lesion scores and oocysts shedding in the faeces was observed.

The invention will now be illustrated by means of the following examples which should in no way be interpreted as limiting the embodiments thereof.

### Example 1 - influence of an ibuprofen - arginine combination and some L-arginine compounds on lesion scores and number of oocysts in faecal samples after an experimental induced coccidiosis infection.

For each experimental group, 30 one day-old male Ross broiler chickens with an average weight of 40 ± 5 g were housed in rooms with constant lighting and a temperature maintained between 25 and 28 °C. Feed of a composition given in Table 1 without anti-coccidials was available *ad libitum.*

**Table 1**

| Item | % |
|---|---|
| Maize | 32,5 |
| Wheat | 40 |
| Soybean | 13,27 |
| Animal fat | 5 |
| Dicalcium phosphate | 0,66 |
| Limestone | 3,25 |
| Feed chalk | 4 |
| Laying core | 1 |
| Sodium chloride | 0,19 |
| D,L-methionine | 0,115 |
| L-Lysine HCl | 0,01 |
| Red and yellow carophylle | 0,005 |

For each group, a drinking water formulation was provided as follows:
- group 1 received a drinking water formulation comprising a combination of ibuprofen and arginine prepared as follows: a mixture of equivalent amounts of ibuprofen and arginine and 2 % citric acid were granulated with a 10 % (weight/weight) Tween 60® surfactant solution. Tween may be added in a suitable amount, e.g. between 5 and 50% w/w. The granulate was dried at room temperature during 24 hours, then a portion thereof was dissolved in drinking water and a dose of the dissolved granulate corresponding to 15 mg Ibuprofen per kg bodyweight per day in water was administered,
- group 2 received a dissolved granulate of L-arginine free base (granulated with a 25 % (weight/volume) Tween 60® surfactant solution, followed by drying at room temperature during 24 hours) corresponding to 15 mg per kg bodyweight in water,
- group 3 received a water solution of L-arginine hydrochloride corresponding to 15 mg per kg bodyweight, and
- groups 4 (infected but untreated chickens) and 5 (uninfected chickens) received drinking water without additives.

Experimental infections were initiated at the age of 3 weeks in each chicken of groups 1 to 4 by administering a designated number of oocysts in 1 ml water into the crop. *Eimeria* species used for infections were obtained from faeces and intestines content of infected chickens and stored in a 2.5% potassium bichromate solution at a temperature of 4 ± 2 °C till use. Just before use, the infecting materials were rinsed with a saturated salt solution (31.7 g NaCl/100 ml water) and water to remove faeces, intestinal compounds and potassium bichromate. After rinsing, the inoculation liquid contained 10⁴ *E. tenella,* 1.5 x 10⁴ *E. acervulina* and 10³ *E. maxima* in 1 ml water.

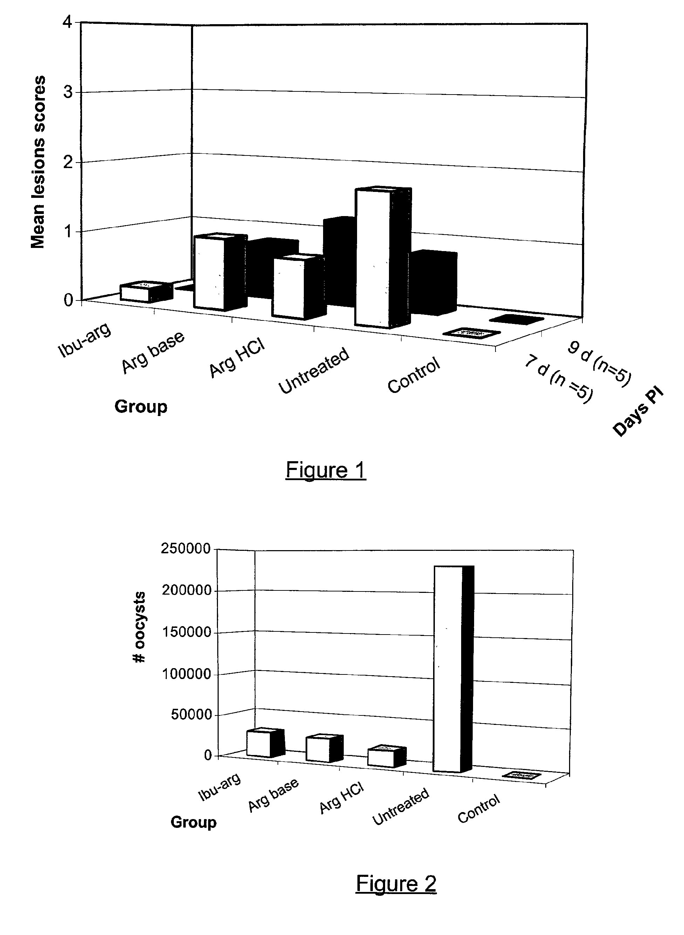

Five chickens of each group were dissected 7 days and 9 days post inoculation (PI) respectively in order to score the intestinal lesions by using the method of Johnson and Reid (referred *supra).* Microscopic sections of the intestines were performed in order to identify the species and to confirm the results of the scoring method as represented in figure 1 which shows a significant decrease in the lesions scores on 7 days post inoculation (PI) between the arginine treated groups 1 to 3 and the untreated infected group 4.

Faecal samples were collected from 5 chickens of each group from days 5 to 9 post inoculation. The number of oocysts was counted by using a McMaster type chamber and expressed per gram faeces and is represented in figure 2 showing a significant decrease in the number of oocysts between the arginine treated groups 1 to 3 and the untreated infected group 4.

### Example 2 - influence of the administration of a ibuprofen- arginine combination and of L-arginine hydrochloride in a drinking water medication on lesion scores after a challenge infection with 2 x 10⁵ E.acervulinaoocysts following a limited primary infection.

For each experimentatal group, 15 one day-old male Ross broiler chickens with average weight of 40 ± 5 g were housed in rooms with constant lighting and a temperature maintained between 25 and 28 °C. Feed without anticoccidials was available *ad libitum* as in example 1. For each group, a drinking water formulation was provided as follows:
- groups 1 and 2 received a dissolved granulate of a combination of ibuprofen and arginine prepared as in example 1 but in a dose of respectively 25 and 50 mg Ibuprofen per kg bodyweight,
- groups 3 and 4 received a drinking water solution of L-arginine hydrochloride in a dose of respectively 500 and 1000 mg per kg bodyweight, and
- groups 5 (infected but untreated chickens) and 6 (uninfected chickens) received drinking water without additives.

Experimental infections were initiated at the age of 3 weeks in each chicken of group 1 to 5 by administering a designated number of *E. acervulina* oocysts in 1 ml water into the crop. *E. acervulina* used for the infection was obtained from intestines content of infected chickens and stored in a 2.5% potassium bichromate solution at a temperature of 4 ± 2 °C till use. Just before use, the infecting materials were rinsed with a saturated salt solution (31.7 g NaCl/100 ml water) and water to remove intestinal compounds and potassium bichromate. After rinsing, the inoculation liquid contained 2 x 10⁵ *E. acervulina* in 1 ml water.

Ten chickens of each group were dissected 7 days post inoculation (PI) to score the intestinal lesions by using the method of Johnson and Reid (referred *supra*). Microscopic sections of the intestines are performed to identify the species and to confirm the results of the scoring method as represented in Fig. 3.

E. acervulina doses of 2 x 10⁵ oocysts in 1 ml water were administered to obtain lesion scores of about 2-2.5. For the infected groups, lower lesions scores were obtained firstly due to a limited primary infection in the untreated infected group - where the chickens have gained some immunity - and secondly due to the preventive administration of the treatments. As shown in figure 3, there is a significant difference in lesion scores between the untreated group and the treated groups. Due to a limited primary infection - as shown by the higher than expected score (zero) of *E. acervulina* lesions in the control group - the untreated groups have gained some immunity against *E. acervulina.* The treated groups did not gained any immunity as they were not primarly infected due to the preventive treatment. Due to the immunity developed after a primary infection, the chickens in the untreated infected group showed a lower lesion score compared with the treated groups where no primary infection has occurred.

### Example 3 - Administration of L-arginine to laying hens to study the influence on the onset of laying eggs and on the weight of layed eggs

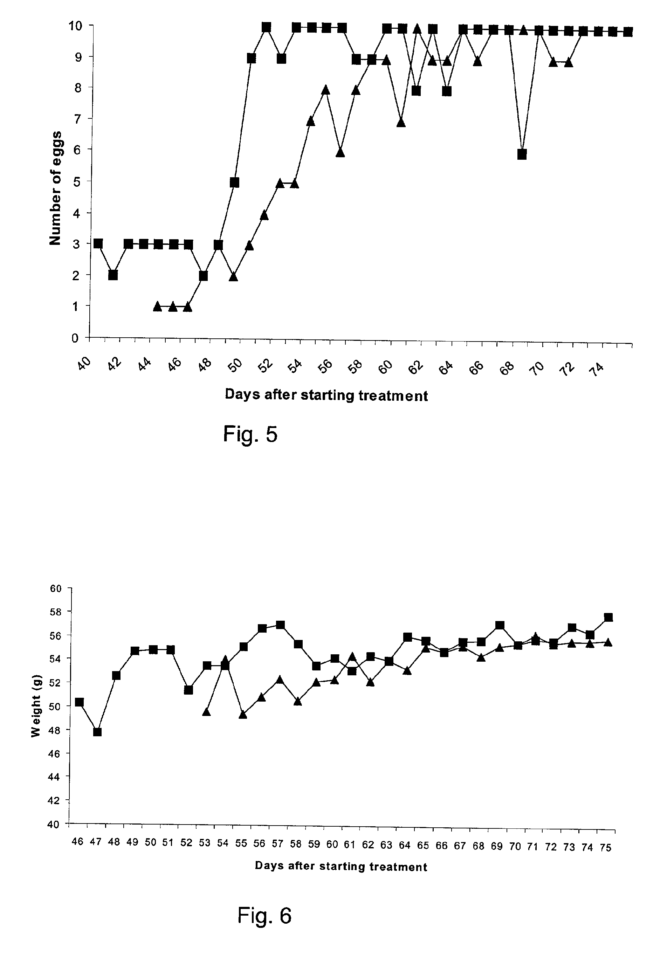

The end of the development of the reproductive organ of laying hens starts on the age of 15 weeks and continues during 7 weeks. 10 laying hens were treated with L-arginine via drinking water from the age of 15 weeks till the age of 21 weeks and 10 laying hens were used as a control group. L-arginine was dosed at 15 mg per kg weight per day. L-arginine free base or a L-arginine salt such as hydrochloride may be given daily in a dose of about 5 - 200 mg up to 5 to about 1000 mg per kg bodyweight per day. Light schemes were taken into account: 8 hours light at the age of 15 and 16 weeks old, 9 hours at 17 weeks, 10 hours at 18 weeks, 11 hours at 19 weeks, 12 hours at 20 weeks and 13 hours at 21 weeks old. The treatment with L-arginine was started on the age of 15 weeks. After 40 days of treatment, 3 of 10 chickens treated with L-arginine started laying while one chicken of the control group started laying four days later (Fig. 5). It is unexpected that the onset of egg-laying is earlier than in the control group which is a commercially important aspect of the present invention. The average weight of the eggs (number of eggs ≥ 3) was above 50 g after 46 days in the treated group while in the control group after 54 days the average weight of at least 3 eggs was above 50 g (Fig 6). Again this improvement is unexpected and is of commercial value.

## Claims

1. Use of a basic amino-acid or amino-acid derivative in the feed and/or drinking water of vertebrates for the prophylactic treatment of a parasitic infection of a vertebrate with a protozoan protist.

2. Use of a basic amino-acid or amino-acid derivative for the manufacture of a feed composition and/or drinking water composition for the prophylactic treatment of a parasitic infection with a protozoan protist in a vertebrate.

3. Use of a basic amino-acid or amino-acid derivative for the manufacture of a medicine for the prophylactic treatment of a parasitic infection with a protozoan protist in a mammal.

4. Use according to any of the claims 1 to 3, further including the use of a combination of a non-steroidal anti-inflammatory drug and of the basic amino-acid or amino-acid derivative.

5. Use according to any of the claims 1 to 4 further comprising the use of a combination of a modulator which stimulates the NO synthesis pathway and of the basic amino-acid or amino-acid derivative.

6. A feed and/or drinking water composition for vertebrates comprising an antiprotozoal prophylactively effective amount of a basic amino-acid or amino-acid derivative and optionally an alimentary acceptable carrier.

7. A veterinary composition for vertebrates comprising an antiprotozoal prophylactively effective amount of a basic amino-acid or amino-acid derivative and optionally a veterinary acceptable carrier.

8. A pharmaceutical composition comprising an antiprotozoal prophylactively effective amount of a basic amino-acid or amino-acid derivative and optionally a pharmaceutically acceptable carrier.

9. A composition according to any of claims 6 to 8, further comprising a prophylactively effective amount of a modulator which stimulates the NO synthesis pathway.

10. A composition according to any of claims 6 to 8, further comprising a prophylactively effective amount of a non-steroidal anti-inflammatory drug and respectively, a pharmaceutically, veterinary or an alimentary acceptable carrier.

11. A composition according to any of claims 6 to 10, in the form of a formulation to be used in drinking water.

12. A composition according to any of claims 6 to 11, wherein the antiprotozoal prophylactively effective amount is an oocyst production limiting amount.

13. A composition according to any of claims 6 to 11, wherein the antiprotozoal prophylactively effective amount is an amount which stimulates the production of reactive oxygen intermediates and/or reactive nitrogen intermediates in the body of the vertebrate to which the composition is administered.

14. A composition according to any of claims 6 to 11, wherein the antiprotozoal prophylactively effective amount of the basic amino acid or the amino acid derivative is sufficient as a substrate for nitrogen oxide synthetase.

15. A composition according to any of claims 6 to 11, wherein the antiprotozoal prophylactively effective amount is sufficient to stimulate a nitrogen oxide synthesis pathway.

16. A use according to any of the claims 1 to 5 or a composition according to any of claims 6 to 15, wherein the basic amino-acid is a naturally-occurring basic amino-acid.

17. A use according to any of the claims 1 to 5 or 16 or a composition according to any of claims 6 to 16, wherein the basic amino-acid is arginine.

18. A use according to a claim 4 or a composition according to claim 14, wherein the non-steroidal anti-inflammatory drug is an acid.

19. A use or a composition according to claim 18, wherein the combination is a salt of the acid non-steroidal anti-inflammatory drug and of the basic amino-acid.

20. A use or composition according to claim 18, wherein the combination is the arginine or lysine salt of ibuprofen.

21. A use according to any of the claims 1 to 5 or 16 to 20 or a composition according to any of the claims 6 to 20, wherein the parasitic infection is coccidiosis.

22. The use of a basic amino acid or a derivative thereof in a drinking water formulation for the enhancement of egg production in poultry.

23. A drinking water composition comprising a basic amino acid or a derivative thereof for the enhancement of egg production in poultry.
